# EUROPÄISCHE PATENTSCHRIFT

(11) **EP 0 800 835 B1**
(45) Veröffentlichungstag und Bekanntmachung des Hinweises auf die Patenterteilung: **10.07.2002**
(21) Anmeldenummer: 97106025.6
(22) Anmeldetag: 11.04.1997
(51) Int. Cl.: A61B 1/12, A61M 3/02, A61M 1/00

(54) **Gerät zum Spülen von Körperhöhlen**
Device for the irrigation of body cavities
Appareil d'irrigation des cavités du corps humain

(30) Priorität: 11.04.1996 DE 29606600 U
(43) Veröffentlichungstag der Anmeldung: 15.10.1997
(73) Patentinhaber: WISAP GESELLSCHAFT FÜR WISSENSCHAFTLICHEN APPARATEBAU MBH, D-82054 Sauerlach (DE)
(72) Erfinder: Semm, Horst Karl, 82031 Grünwald (DE)
(74) Vertreter: Heim, Hans-Karl, Dipl.-Ing.

(56) Entgegenhaltungen:
- EP-A- 0 307 895
- WO-A-86/03361
- WO-A-87/04913
- WO-A-88/07384
- US-A- 4 585 436
- US-A- 5 368 569

## Beschreibung

Die Erfindung betrifft ein Gerät zum Spülen von Körperhöhlen mit einer Flüssigkeit, insbesondere zum Spülen mit physiologischer Kochsalzlösung bei Laparotomien, Pelviskopien und Laparoskopien, gemäß dem Oberbegriff des Anspruchs 1.

Ein derartiges Gerät findet während therapeutischer und chirurgischer Eingriff Verwendung, um eine Wunde mit einer Spülflüssigkeit durch Waschen zu reinigen. Danach wird die mit Blut, Geweberesten und anderen Partikeln versetzte Flüssigkeit abgesaugt.

Für endoskopische Bauch- oder Beckenspülungen, die während oder nach einer operativ-therapeutischen Pelviskopie oder Laparoskopie erforderlich werden können, wird in der Regel physiologische Kochsalzlösung eingeführt, welche auf Körpertemperatur von etwa 37 - 38° C erwärmt ist.

Diese Spülflüssigkeit wird in Kunststoffbeuteln in einem Aufnahmeraum derart aufgehängt, daß ein rückseitiger Kontakt zu einer Wärmeplatte gegeben ist. Ein Schlauchsystem zur Irrigation der Flüssigkeit bei einem vorwählbaren Druck wird in einem unteren Bereich angeschlossen, während eine gerätetechnische Einheit mit einer Temperaturregelung und Drucksteuerung sowie einer Pumpe in einem Geräteteil oberhalb des Aufnahmeraums angeordnet ist. Anzeigeinstrumente, insbesondere eine Vorwahldruckanzeige und eine Temperaturanzeige ermöglichen eine Kontrolle der Irrigationsparameter.

Die vertikale Anordnung des Spülflüssigkeitsbeutels im Aufnahmeraum verhindert eine rasche Erwärmung und einen effizienten Wärmeübergang von der Wärmeplatte zur Spülflüssigkeit. Um diesen Nachteilen entgegenzuwirken und die Verlustenergie infolge einer unzureichenden Anlage des Beutels an der Wärmeplatte zu minimieren, werden meist zusätzliche Haltemittel vorgesehen. Diese sollen eine möglichst großflächige Anlage des meist kissenartigen Spülflüssigkeitsbeutels an der Wärmeplatte bewirken.

Aus der WO 86/03361 ist ein peritonales Dialysegerät mit einer Heizeinrichtung zur Aufnahme von Flüssigkeitsbeuteln, welche auf einen vorgegebenen Wert erwärmt und gewogen werden, bekannt. Die Heizeinrichtung weist eine konkave Platte auf, welche in einem quaderförmigen Gehäuse angeordnet und mit einer Kraftmessdose verbunden ist. Ein Temperatursensor in der Heizplatte misst die Temperatur des Flüssigkeitsbeutels und ein weiterer Temperatursensor ist zur kontinuierlichen Messung der Heizplatte angeordnet. Eine elektronische Schaltung gewährleistet die Einhaltung einer vorgebbaren Temperatur der Flüssigkeit.

In der WO 87/04913 ist ein Gerät zum Spülen von Körperhöhlen beschrieben, welches außerordentlich kompakt ausgebildet ist. Ein Flüssigkeitsbeutel wird in einer Druckkammer angeordnet, welche ein aufblasbares Element und eine verschließbare Kammertür aufweist. Eine Heizeinrichtung für die Spülflüssigkeit ist nicht vorgesehen, und Bedien- und Schaltelemente sind in einer nahezu horizontalen Deckfläche des Gerätes aufgenommen, wodurch eine einfache Bedienung und Kontrolle der Anzeigeelemente bzw. Schalter nicht immer gewährleistet ist.

Bei einem aus der US-PS 4,585,436 bekannten Gerät zur automatisierten peritonalen Dialyse ist der Dialyseapparat als ein unteres Geräteteil und zur Aufnahme eines Flüssigkeitsbeutels, einer Pumpe und eines verzweigten Schlauchsystems mit einem X-Verbinder und Absperreinrichtungen für eine vorgebbare Flüssigkeitszu- und -abführung ausgebildet sowie mit einer Wägeeinrichtung zur Bestimmung des abgezogenen bzw. eingeführten Flüssigkeitsvolumens versehen. Oberhalb des unteren Geräteteils sind an einem Gestell eine Heizeinrichtung, welche zur Aufnahme eines Beutels mit Dialyseflüssigkeit dient und konkav ausgebildet ist, und eine Halterung für mehrere Vorratsbeutel mit Dialyseflüssigkeit angeordnet. Aufgrund dieser Konstruktion muss das untere Geräteteil sehr kompakt und stabil ausgebildet sein, damit die erforderliche Stabilität des nach oben auskragenden Gestells mit der nahezu horizontal angeordneten Heizeinrichtung und den Vorratsbeuteln gewährleistet ist. Außerdem erfordert das gesamte Gerät einen relativ großen Platzbedarf, und durch die Anordnung der Schalter und Anzeige- bzw. Messgeräte im unteren Geräteteil ist die Bedienung und Kontrolle relativ ungünstig.

Der Erfindung liegt die **Aufgabe** zugrunde, ein Gerät zum Spülen von Körperhöhlen, insbesondere für endoskopische Untersuchungen und Eingriffe, zu schaffen, welches unter ergonomischen und ökonomischen Aspekten mit einem besonders geringen konstruktiven Aufwand und Energieaufwand eine wirkungsvolle Erwärmung einer Spülflüssigkeit auf eine vorgesehene Temperatur und deren Aufrechterhaltung sowie eine einfache Handhabung und die erforderliche Stabilität des Gerätes ermöglicht.

Erfindungsgemäß wird die Aufgabe durch die Merkmale des Anspruchs 1 gelöst. Zweckmäßige und vorteilhafte Ausgestaltungen sind in den Unteransprüchen und in der Figurenbeschreibung enthalten.

Ein Grundgedanke der Erfindung kann darin gesehen werden, in einem gemeinsamen, pultartig ausgebildeten Gehäuse ein oberes Geräteteil, in welchem eine Wärmeplatte zur Auflage wenigstens eines Spülflüssigkeitsbeutels angeordnet ist, und ein unteres Geräteteil, in welchem gerätetechnische Bauteile angeordnet werden, aufzunehmen. Die pultartige Ausbildung wird erfindungsgemäß durch einen definierten Neigungswinkel der Gehäusefrontseite und der darin angeordneten Wärmeplatte und durch eine vertikale Ausrichtung des unteren Geräteteils erreicht.

Erfindungsgemäß sind bei einem Gerät zum Spülen von Körperhöhlen mit einer Flüssigkeit, insbesondere zum Spülen mit physiologischer Kochsalzlösung bei Laparatomien, Pelviskopien und Laparoskopien, welches eine schräg angeordnete Wärmeplatte zur Auflage wenigstens eines Spülflüssigkeitsbeutels in einem oberen Geräteteil und gerätetechnische Bauteile zur Beeinflussung von Druck und Temperatur der Spülflüssigkeit in einem unteren Geräteteil aufweist, das obere Geräteteil und das untere Geräteteil in einem Gehäuse aufgenommen, welches pultartig ausgebildet ist, dergestalt, dass das Gehäuse im Bereich des unteren Geräteteils vertikal ausgerichtet ist und dass der Neigungswinkel α zwischen der Gehäusefrontseite im Bereich des oberen Geräteteils, in welcher die Wärmeplatte wärmeisoliert angeordnet ist, und einer lotrechten Vertikalen, 30 +/- 15° beträgt.

Durch den Neigungswinkel α ist gewährleistet, daß ein Spülflüssigkeitsbeutel im wesentlichen ohne zusätzliche Halterung auf der Wärmeplatte angeordnet werden kann. Es genügt ein Einhängen an einem hakenähnlichen Befestigungselement, welches beispielsweise verschiebbar an einem Ständer oberhalb des oberen Gehäuseteils angeordnet ist.

Eine Gehäuserückseite des oberen Gehäuseteils kann in einer besonders vorteilhaften Geräteausbildung parallel zu der geneigten Wärmeplatte und der Frontseite des oberen Geräteteils angeordnet sein. Es besteht auch die Möglichkeit, die Rückseite des oberen Geräteteils nahezu lotrecht auszubilden.

Es ist vorteilhaft, dass die Wärmeplatte in dem pultartigen Gehäuse oberhalb des unteren Geräteteils, in welchem wenigstens eine Temperaturregelung und eine Drucksteuerung, zweckmäßigerweise auch eine Pumpe für die Spülflüssigkeit angeordnet sind, vorgesehen ist.

Auf der geneigten Wärmeplatte liegt ein Spülflüssigkeitsbeutel mit seiner rückseitigen Fläche aufgrund des Eigengewichtes der Spülflüssigkeit nahezu auf und gewährleistet durch einen großflächigen Kontakt einen effizienten Wärmeübergang. Die Temperatur eines vorgewärmten Spülflüssigkeitsbeutels, welcher auf der Wärmeplatte aufliegt, wird mit geringem Energieaufwand konstant gehalten. In der Regel werden vorgewärmte Spülflüssigkeitsbeutel verwendet. Ein nicht vorgewärmter Spülflüssigkeitsbeutel kann aufgrund des großflächigen Kontaktes zwischen Wärmeplatte und Spülflüssigkeit jedoch ebenfalls relativ rasch auf Körpertemperatur, d. h. auf eine Temperatur im Bereich von 37 bis 38° C, erwärmt werden.

Neben einer effizienten Erwärmung und Temperaturkonstanthaltung einer Spülflüssigkeit wirkt sich eine frontseitige Schräganordnung des Gehäuses des oberen Geräteteils vorteilhaft auf eine damit erreichbare, besonders flache Gerätekonstruktion aus. Der Platzbedarf des Gerätes ist äußerst gering und vermittelt einen außerordentlich vorteilhaften Gesamteindruck, insbesondere wenn nicht nur das gesamte obere Geräteteil pultartig schräg nach hinten verlaufend ausgebildet ist, sondern auch die Rückseite des oberen Geräteteils parallel zur Frontseite des oberen Geräteteils ausgebildet ist. Mit schräg nach innen gerichteten Seitenflächen am oberen und gegebenenfalls auch am unteren Geräteteil wird eine weitere Reduzierung der Raummaße erreicht.

Das untere Geräteteil, in welchem insbesondere Regelungseinheiten und die weitere Gerätetechnik aufgenommen sind und welches beispielsweise als ein weitgehend rechteckiger Gehäusebereich ausgebildet sein kann, verlagert den Schwerpunkt des gesamten Gerätes nach unten, was besonders vorteilhaft für eine Halterung und Fixierung des Gerätes an einem stativartigen Träger und die erforderliche Stabilität des Gerätes ist.

Der vorteilhafte Gesamteindruck eines relativ flachen und leichten Gerätes wird dadurch unterstützt, daß aufgrund der definierten Neigung der Wärmeplatte und der Gehäusefrontseite des oberen Geräteteils eine Abdeckung oder eine gitterähnliche Halterung vor der Wärmeplatte entfallen kann.

Es genügt eine Fixierung des Spülflüssigkeitsbeutels , z.B. mit einem oberen Randbereich an einem Stativträger mit Hilfe eines Befestigungselementes.

Besonders vorteilhaft ist eine automatische Aufheizung der Wärmeplatte, sobald ein Spülflüssigkeitsbeutel auf der Wärmeplatte aufliegt. Zu diesem Zweck ist vorzugsweise in einem unteren Bereich der Wärmeplatte ein Sensorbereich mit wenigstens einer Sensoreinrichtung, z. B. einem Wärmesensor, angeordnet, welcher vorzugsweise ein optoelektronisches Bauelement ist und nach dem Echolotprinzip arbeitet. Der Wärmesensor detektiert einen aufliegenden Spülflüssigkeitsbeutel und aktiviert eine Heizungsregelung einer Heizeinrichtung. Es sind zum Detektieren auch Bewegungssensoren geeignet. Vorteilhaft kann eine Fotodiode verwendet werden, welche bevorzugt in einer mittigen Vertiefung des Sensorbereiches angeordnet wird.

Es ist zweckmäßig, den Sensorbereich als eine nahezu kreisrunde Metallplatte und wärmeisoliert sowie bevorzugt mit einem geringfügigen Überstand in der Wärmeplatte anzuordnen. Um unverfälschte Meßergebnisse zu erhalten, ist es erfindungsgemäß vorgesehen, die Sensorbereich-Platte gegen die Wärmeplatte zu isolieren und z.B. mit einem relativ breiten, ringförmigen Wärmeisolator, z. B. aus einem Kunststoffmaterial, zu versehen.

Im Bereich des Wärmesensors sind auf dem plattenförmigen Sensorbereich zweckmäßigerweise Sicherheitsthermostaten als Thermoschalter angeordnet, welche die Heizung steuern und bei Überhitzung abschalten. Vorteilhafterweise sind zwei Sicherheitsthermostaten bevorzugt unterhalb und seitlich versetzt zum Wärmesensor auf der Rückseite des Sensorbereichs befestigt, beispielsweise angeklebt.

Die Erwärmung der Wärmeplatte, welche z.B. aus Aluminium bestehen kann, erfolgt von der Rückseite her. Bevorzugt sind Heizfolien mit beispielsweise mäanderförmig aufgebrachten Leiterbahnen angeordnet. Eine optimale Erwärmung eines auf der Vorderseite aufliegenden Spülflüssigkeitsbeutels wird z.B. erreicht, wenn drei etwa rechteckige Heizfolien vorgesehen sind, von denen zwei beidseitig des Sensorbereichs von nahe einer unteren Kante in Längsrichtung angeordnet sind und die dritte Heizfolie mit geringem Abstand zu den längs ausgerichteten, unteren zwei Heizfolien quer zu diesen angeordnet wird. Dabei bleibt ein oberer Bereich der rückseitigen Heizplatte frei. Eine flächenhafte Erwärmung der Spülflüssigkeit des aufliegenden Beutels wird aufgrund der Wärmeleitung der Wärmeplatte in Richtung Oberkante gewährleistet.

Der untere Teil des Gerätes, welcher bevorzugt rechteckig ausgebildet ist, enthält die Temperaturregelung und Drucksteuerung, die Pumpe, sowie Schalter, beispielsweise für einen "Schaukelprozeß" zur Entlüftung des Pumpen- und Schlauchsystems, und Anzeigen, insbesondere für die Temperatur der Spülflüssigkeit und für den Spüldruck. Die Temperaturanzeige kann jedoch ebenso im oberen Geräteteil angeordnet sein.

Als Pumpe kann zweckmäßigerweise eine Kreiselpumpe verwendet werden, welche einen geräteseitigen Antriebsteil und einen auf diesen aufsteckbaren Pumpenkopf aufweist. Diese Flüssigkeitspumpe ist grundsätzlich für ein Gerät zum Spülen von Körperhöhlen geeignet und nicht auf eine pultartige Gehäuseausbildung begrenzt.

Das untere Geräteteil und das obere Geräteteil mit der Anordnung der Wärmeplatte im frontseitigen Gehäusebereich und einem Sensorbereich für einen aufliegenden Spülflüssigkeitsbeutel bilden ein pultartiges Gerät, welches sich vorteilhaft von den bekannten kompakten Geräten unterscheidet.

Durch die pultartige Ausbildung des gemeinsamen Gehäuses für das untere und obere Geräteteil und den definierten Neigungswinkel der Heizplatte bzw. der Gehäusefrontseite des oberen Geräteteils wird eine einfache und gleichzeitig optimale Auflage eines Beutels mit der zu erwärmenden Spülflüssigkeit erreicht. Die vertikale Ausrichtung des unteren Geräteteils mit den darin aufgenommenen Geräten, Bedienund Anzeigelementen gewährleistet zusammen mit der schrägen Ausbildung des oberen Geräteteils eine besonders flache Gerätekonstruktion, welche mit einem außerordentlich geringen Platzbedarf verbunden ist. Gleichzeitig wird durch das untere, vertikal ausgerichtete Geräteteil eine Verlagerung des Schwerpunktes des gesamten Gerätes nach unten erreicht, was sich für die Stabilität und die Befestigung des Gerätes an einem stativartigen Träger vorteilhaft auswirkt. Das Gerät kann in der jeweils erforderlichen Höhe angeordnet werden, wodurch die Bedienung und Kontrolle optimiert werden können.

Die Erfindung wird nachstehend anhand eines Ausführungsbeispiels weiter erläutert. In der zugehörigen Zeichnung zeigen in einer stark schematisierten Darstellung
- Figur 1: ein erfindungsgemäßes Gerät in einer perspektivischen Darstellung;
- Figur 2: eine optische Temperaturanzeige des Gerätes und
- Figur 3: eine Draufsicht auf die Wärmeplatte mit Sensorbereich.

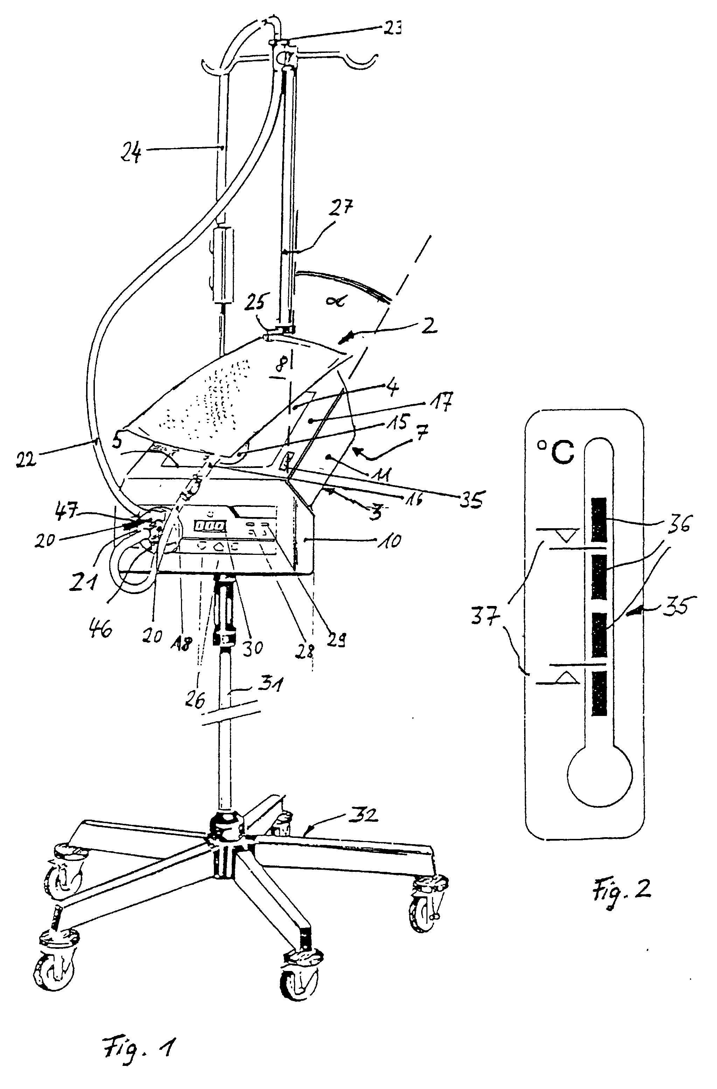

Das Gerät 2 gemäß Figur 1 ist pultartig aufgebaut und weist ein Gehäuse 7 für ein oberes Geräteteil 11 und ein unteres Geräteteil 10 auf.

Während das untere Geräteteil 10 im wesentlichen vertikal angeordnet ist, bildet das obere Geräteteil 11 durch eine schräg nach hinten geneigte Anordnung eine Auflage für einen Spülflüssigkeitsbeutel 8. Dieser Spülflüssigkeitsbeutel 8 liegt auf einer Wärmeplatte 4 einer Gehäusefrontseite 17 des oberen Geräteteils 11 großflächig auf, da die Gehäusefrontseite 17 und die Wärmeplatte 4 mit einem Neigungswinkel α im Bereich von 30° +/- 15° angeordnet ist.

Durch die nach oben und zu einer Rückseite 3 geneigte Anordnung der Wärmeplatte 4 wird eine ganzflächige Auflage des Spülflüssigkeitsbeutels 8 mit seiner Beutelrückseite auf der Wärmeplatte 4 erreicht. Damit verbunden ist ein effizienter Wärmeübergang zwischen der Wärmeplatte 4 und der Spülflüssigkeit, so daß durch die konstruktive Ausbildung der geneigten Wärmeplatte 4 eine besonders effiziente Aufheizung und/oder Temperaturkonstanthaltung der Spülflüssigkeit gewährleistet ist. Zweckmäßigerweise entspricht die Größe und geometrische Form der Wärmeplatte 4 der rückseitigen Auflagefläche des Spülflüssigkeitsbeutels 8, welcher prinzipiell kissenartig ausgebildet ist.

Der Neigungswinkel von etwa 30°, mit welchem das obere Geräteteil 11 nach hinten geneigt ist, ermöglicht eine einfache und unkomplizierte Aufhängung des Spülflüssigkeitsbeutels 8 an einem Befestigungselement 25. Dieses kann als ein Aufnahmering 25 mit Haken an einer Haltestange 27 oberhalb des Gerätes 2 und drehbar an der Haltestange 27 angeordnet sein. Vorteilhaft sind mehrere, auch unterschiedlich lange Haken für Spülflüssigkeitsbeutel 8 unterschiedlicher Volumina, z. B. von 1 bis 5 l, vorgesehen.

In einem unteren Bereich des Spülflüssigkeitsbeutels 8 ist ein Primärschlauch 18 mit einem Einstechdorn 16 eingeführt. Der Primärschlauch 18 führt zu einer Flüssigkeitspumpe 20. Vom Ausgang der Pumpe 20 führt ein Sekundärschlauch 22 zu einem Schlauchadapter 23, an welchem ein zu einem Saug-/Spülinstrument 13 führender Spülschlauch 24 angeschlossen ist.

Das Saug-/Spülinstrument 13 weist monofil-bivalente Spülrohre auf und erlaubt alternativ die Steuerung der Instillationsmenge von auf Körpertemperatur vorgewärmter Kochsalzlösung oder ihr Absaugen mit Blut- bzw. Gewebepartikln über ein kontaminationsfreies Absaugsystem.

Bevor das Gerät 2 zum Spülen eingesetzt wird, werden die Schläuche 18, 22 und insbesondere die Flüssigkeitspumpe 20 entlüftet. Eingeschlossene Luft vermindert die Leistungsfähigkeit der Pumpe. Vorteilhafterweise ist bei dem Gerät 2 ein "Schaukelprozeß" zur Entlüftung des Pumpen- bzw. Schlauchsystems installiert, welcher durch Druckvorwahltasten 26. im unteren Geräteteil 10 ausgelöst werden kann und z.B. ein Umschalten zwischen Werten von 0 und 150 mm Hg-Säule vorsieht. Der Spüldruck des nachfolgenden Spülprozesses beträgt in der Regel automatisch 150 mm Hg-Säule. Er kann jedoch vorgewählt und über die Druckvorwahltasten 26 in einen Bereich zwischen 50 und 310 mm Hg-Säule eingestellt werden. Eine Digitalanzeige 30 gibt den eingestellten Ist-Wert des Insufflationsdruckes wieder.

Eine Heizungsregelung für die Wärmeplatte 4 wird durch einen auf dieser aufliegenden Flüssigkeitsbeutel 8 automatisch aktiviert. Zu diesem Zweck weist die Wärmeplatte 4 nahe einer unteren Kante 5 einen Sensorbereich 15 auf, welcher in diesem Ausführungsbeispiel als kreisförmige VA-Platte ausgebildet und durch einen relativ breiten Isolationsring 6 wärmeisoliert gegen die Wärmeplatte 4 angeordnet ist.

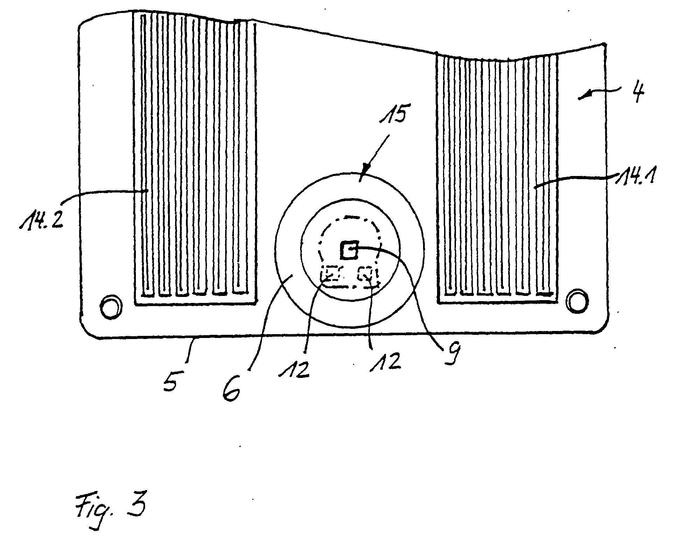

Gemäß Figur 3 weist der Sensorbereich 15 wenigstens einen Sensor, insbesondere einen Wärmesensor 9, als sogenannten "Beutelsensor" auf, welcher zum Detektieren eines aufliegenden Flüssigkeitsbeutels 8 und zum Aktivieren einer Heizungsregelung dient.

Die kreisförmige Metallplatte des Sensorbereichs 15 ist in der etwa kreisringförmigen Isolation 6 aufgenommen und steht mit dieser geringförmig über die Wärmeplatte 4 über. Der mittig angeordnete Wärmesensor 4, welcher bevorzugt auf optischer Basis und nach dem Echolotprinzip arbeitet, ist in einer Vertiefung des Sensorbereichs 15 angeordnet.

Auf der Rückseite des plattenförmigen Sensorbereichs 15 sind zwei Sicherheitsthermostaten 12, welche die Temperatur des Spülflüssigkeitsbeutels 8 kontrollieren und konstant halten sowie bei Überhitzung abschalten, angeordnet. Diese Thermoschalter 12, welche mit der Heizungsregelung verbunden sind, sind in Fig. 3 mit strichlierten Linien dargestellt. Sie sind unterhalb des Wärmesensors 9 und seitlich versetzt zu diesem in einer vorgesehenen Ausnehmung des rückseitigen Bereichs des Isolationsrings 6, welcher strichpunktiert dargestellt ist, angeordnet.

Figur 3 verdeutlicht außerdem die Anordnung der Heizeinrichtung 14 auf der Rückseite der Wärmeplatten mit zwei in Längsrichtung ausgerichteten Heizfolien 14.1 und 14.2 beidseitig des Sensorbereichs 15. Eine dritte Heizfolie oberhalb und quer zu den Heizfolien 14.1 und 14.2 ist aus der ausschnittsweise dargestellten Wärmeplatte 4 der Fig. 3 nicht erkennbar.

Die Wärmeplatte 4 ist im Bereich der geneigten Gehäusefrontseite 17 des Geräteoberteils 11 angeordnet (s. Fig. 1). Das gesamte Gerät 2 ist an einem Stativrohr 31, gegebenenfalls auch höhenverstellbar, befestigt. Zweckmäßigerweise ist der Träger 31 mit einem Mehrfuß, z. B. mit einem Fünffuß 32, mit antistatischen Kunststoffrollen versehen.

Im unteren Geräteteil 10 sind Anzeigeelement 28, 29 der Heizungsregelung angeordnet. Das Anzeigeelement 28 ist z. B. eine grün emittierende Leuchtdiode (LED), welche die eingeschaltete Heizungsregelung bei Auflage eines vorgewärmten Spülflüssigkeitsbeutels 8 bei einer Temperatur von ca 34°C anzeigt. Eine zweite grüne LED kann z. B. bei Erreichen einer Temperatur von 37°C leuchten.

Eine rote LED ist als Anzeigeelement 29 angeordnet und schaltet z.B. bei einer Überhitzung von etwa 41°C ein. Zusätzlich kann ein Alarmton vorgesehen sein. Die Heizungsregelung wird abgeschaltet, wenn eine Temperatur von 37°C erreicht ist. Um eine defekte Heizungsregelung anzuzeigen, ist eine blinkende rote LED 29 vorgesehen.

Eine dreistellige 7-Segmentanzeige 30 gibt Auskunft über den vorgewählten Spüldruck. Eine Temperaturanzeige 35 dient der Kontrolle der Temperatur der Spülflüssigkeit im Spülflüssigkeitsbeutel 8. Die Temperaturanzeige 35 sowie weitere Anzeigen können auch im Bereich des oberen Geräteteils 11, insbesondere seitlich des aufliegenden Spülflüssigkeitsbeutels 8 und somit gut erkennbar angeordnet werden.

Das Gerät 2 ist EMV-abgeschirmt ausgebildet und in einem Innenbereich wenigstens des Gehäusedeckels mit einem Kupfer-beschichteten Kunststoffmaterial ausgekleidet. Ein Netzteil wird zusätzlich durch ein entsprechend geformtes Blech EMV-abgeschirmt.

In Figur 2 ist die optische Temperaturanzeige 35 vergrößert dargestellt. Eine Sollbereichsmarkierung 37 und unterschiedliche Farben der Balken LED's 36 ermöglichen ein rasches und sicheres Einordnen des angezeigten Temperaturwertes.

## Patentansprüche

1. Gerät zum Spülen von Körperhöhlen mit einer Flüssigkeit, insbesondere zum Spülen mit physiologischer Kochsalzlösung bei Laparatomien, Pelviskopien und Laparoskopien, mit einer Wärmeplatte (4), welche zur Auflage wenigstens eines Spülflüssigkeitsbeutels (8) schräg in einem oberen Geräteteil (11) angeordnet ist, und mit einem unteren Geräteteil (10), welches gerätetechnische Bauteile zur Beeinflussung von Druck und Temperatur der Spülflüssigkeit aufweist,
**dadurch gekennzeichnet,**
**dass** das obere Geräteteil (11) und das untere Geräteteil (10) in einem Gehäuse (7) aufgenommen sind, welches pultartig ausgebildet ist, dergestalt, dass das Gehäuse (7) im Bereich des unteren Geräteteils (10) vertikal ausgerichtet ist und dass der Neigungswinkel α zwischen der Gehäusefrontseite (17) im Bereich des oberen Geräteteils (11), in welcher die Wärmeplatte (4) wärmeisoliert angeordnet ist, und einer lotrechten Vertikalen 30 +/- 15° beträgt.

2. Gerät nach Anspruch 1,
**dadurch gekennzeichnet,**
**dass** das obere Geräteteil (11) eine Rückseite (3) aufweist und dass die Rückseite (3) parallel zu der mit dem Neigungswinkel α angeordneten Gehäusefrontseite (17) ausgebildet ist.

3. Gerät nach Anspruch 1 oder 2,
**dadurch gekennzeichnet,**
**dass** das untere Geräteteil (10) zur Aufnahme wenigstens einer Temperaturregelung und einer Drucksteuerung ausgebildet ist.

4. Gerät nach einem der vorhergehenden Ansprüche,
**dadurch gekennzeichnet,**
**dass** die Wärmeplatte (4) mit einem Sensorbereich (15) versehen ist, welcher wenigstens einen Sensor (9) zum Detektieren des auf der Wärmeplatte (4) aufliegenden Spülflüssigkeitsbeutels (8) und zum Aktivieren einer Heizungsregelung aufweist.

5. Gerät nach Anspruch 4,
**dadurch gekennzeichnet,**
**dass** der Sensor (9) zum Detektieren des aufliegenden Spülflüssigkeitsbeutels (8) ein Wärmesensor (9) ist und in einer mittigen Vertiefung des Sensorbereichs (15) angeordnet ist.

6. Gerät nach Anspruch 4 oder 5,
**dadurch gekennzeichnet,**
**dass** der Sensorbereich (15) als eine nahezu kreisrunde Metallplatte ausgebildet ist, welche mit einem Isolationsring (6) versehen und in der Wärmeplatte (4) angeordnet ist.

7. Gerät nach einem der Ansprüche 4 bis 6,
**dadurch gekennzeichnet,**
**dass** der Sensorbereich (15) mit einem geringen Überstand in der Wärmeplatte (4) angeordnet ist und als Wärmesensor (9) ein optoelektronisches Bauelement eingesetzt ist, welches nach dem Echolotprinzip arbeitet.

8. Gerät nach einem der Ansprüche 4 bis 7,
**dadurch gekennzeichnet,**
**dass** auf der Rückseite des Sensorbereichs (15) Sicherheitsthermostaten (12) angeordnet sind, welche eine Heizeinrichtung (14) steuern und bei Überhitzung abschalten.

9. Gerät nach Anspruch 8,
**dadurch gekennzeichnet,**
**dass** als Heizeinrichtung (14) Heizfolien auf der Rückseite der Wärmeplatte (4) angeordnet sind, wobei zwei Heizfolien (14.1, 14.2) beidseitig des Sensorbereichs (15) und wenigstens eine weitere Heizfolie oberhalb des Sensorsbereichs (15) und insbesondere quer zu den beidseitig angeordneten Heizfolien (14.1, 14.2) vorgesehen sind.

10. Gerät nach einem der vorhergehenden Ansprüche,
**dadurch gekennzeichnet,**
**dass** die Temperatur der Flüssigkeit im Spülflüssigkeitsbeutel (8) auf Körpertemperatur regelbar und ein Warnsystem bei Überhitzung der Spülflüssigkeit vorgesehen ist.

11. Gerät nach einem der vorhergehenden Ansprüche,
**dadurch gekennzeichnet,**
**dass** eine optische Temperaturanzeige (35) vorgesehen ist, welche Balken-LEDs (36) sowie eine Sollbereichsmarkierung (37) aufweist.

12. Gerät nach einem der vorhergehenden Ansprüche,
**dadurch gekennzeichnet,**
**dass** in dem unteren Geräteteil (10) eine Pumpe (20), Anzeigen (28, 29, 30) und Tasten (26) angeordnet sind, wobei die Tasten (26) Druckwahltasten für einen Schaukelprozeß zum Entlüften der Pumpe (20) und des Schlauchsystems (18, 22) sind.

13. Gerät nach einem der vorhergehenden Ansprüche,
**dadurch gekennzeichnet,**
**dass** die Wärmeplatte (4) aus Aluminium gefertigt ist.

## Claims

1. Apparatus for irrigating body cavities with a liquid, particularly for irrigating with physiological common salt solution in the case of laparotomies, pelviscopies and laparoscopies, having a hotplate (4), which is arranged in inclined manner in an upper apparatus part (11) for supporting at least one irrigating liquid bag (8), and having a lower apparatus part (10), which has apparatus components for influencing the pressure and temperature of the irrigating liquid,
**characterized in that**
the upper apparatus part (11) and the lower apparatus part (10) are received in a casing (7), which has a console-like construction, so that the casing (7) is vertically oriented in the region of the lower apparatus part (12) and the angle of inclination α between the casing front side (17) in the region of the upper apparatus part (11), in which the hotplate (4) is placed in thermally insulated manner, and a perpendicular vertical line is 30 ± 15°.

2. Apparatus according to claim 1,
**characterized in that**
the upper apparatus part (11) has a back (3) and that the back (3) is constructed parallel to the casing front side (17) positioned with the angle of inclination α.

3. Apparatus according to claim 1 or 2,
**characterized in that**
the lower apparatus part (10) is constructed for receiving at least a temperature control and a pressure control.

4. Apparatus according to one of the preceding claims,
**characterized in that**
the hotplate (4) is provided with a sensor area (15), which has at least a sensor (9) for detecting the irrigating liquid bag (8) resting on the hotplate (4) and for activating a heating control.

5. Apparatus according to claim 4,
**characterized in that**
the sensor (9) for detecting the resting irrigating liquid bag (8) is a heat sensor (9) and is located in a central depression of the sensor area (15).

6. Apparatus according to claim 4 or 5,
**characterized in that**
the sensor area (15) is constructed as a virtually circular metal plate, which is provided with an insulating ring (6) and is positioned in the hotplate (4).

7. Apparatus according to one of the claims 4 to 6,
**characterized in that**
the sensor area (15) is placed with a slight projection in the hotplate (4) and as the heat sensor (9) use is made of an optoelectronic component operating according to the echo-sounder principle.

8. Apparatus according to one of the claims 4 to 7,
**characterized in that**
on the back of the sensor area (15) are positioned safety thermostats (12), which control a heating device (14) and switch off in the case of superheating.

9. Apparatus according to claim 8,
**characterized in that**
as the heating device (14) heating foils are placed on the back of the hotplate (4), whereby there being two heating foils (14.1, 14.2) on either side of the sensor area (15) and at least one further heating foil above the sensor area (15) and in particular transversely to the bilaterally positioned heating foils (14.1, 14.2).

10. Apparatus according to one of the preceding claims,
**characterized in that**
the temperature of the liquid in the irrigating liquid bag (8) can be regulated to body temperature and a warning system is provided in the case of superheating of the irrigating liquid.

11. Apparatus according to one of the preceding claims,
**characterized in that**
an optical temperature display (35) is provided, which has bar LEDs (36) as well as a nominal range marking (37).

12. Apparatus according to one of the preceding claims,
**characterized in that**
in the lower apparatus part (10) are positioned a pump (20), displays, (28, 29, 30) and buttons (26), whereby the buttons (26) being push buttons for a rocking process for venting the pump (20) and the hose system (18, 22).

13. Apparatus according to one of the preceding claims,
**characterized in that**
the hotplate (4) is made from aluminium.

## Revendications

1. Appareil pour irriguer les cavités du corps humain avec un liquide, notamment avec une solution de chlorure de sodium physiologique, pour des laparotomies, des pelviscopies et des laparoscopies, comprenant une plaque chauffante (4), installée en biais dans une partie supérieure (11) de l'appareil pour recevoir au moins une poche de liquide d'irrigation (8), et une partie inférieure (10) d'appareil qui comporte les équipements techniques pour agir sur la pression et la température du liquide d'irrigation,
**caractérisé en ce que**
la partie supérieure (11) de l'appareil et la partie inférieure (10) de l'appareil sont logées dans un boîtier (7) en forme de pupitre, de sorte que le boîtier (7) soit aligné verticalement au niveau de la partie inférieure (10) de l'appareil, et que l'angle d'inclinaison α entre une verticale et la face frontale (17) du boîtier au niveau de la partie supérieure (11) de l'appareil dans laquelle est installée la plaque chauffante (4) de manière isolée, fasse 30 ±15°.

2. Appareil, selon la revendication 1,
**caractérisé en ce que**
la partie supérieure (11) de l'appareil comporte une partie arrière (3) et cette partie arrière (3) est parallèle à la face frontale (17) du boîtier faisant l'angle d'inclinaison α.

3. Appareil, selon la revendication 1 ou 2,
**caractérisé en ce que**
la partie inférieure (10) de l'appareil est conçue pour recevoir au moins un moyen de régulation de température et une commande de pression.

4. Appareil, selon l'une des revendications précédentes,
**caractérisé en ce que**
la plaque chauffante (4) comporte une zone de capteurs (15) munie d'au moins un capteur (9) pour détecter la poche de liquide d'irrigation (8) placée sur la plaque chauffante (4) et pour activer une régulation de chauffage.

5. Appareil, selon la revendication 4,
**caractérisé en ce que**
le capteur (9) pour détecter la poche de liquide d'irrigation (8) est un capteur thermique (9) et est installé dans une cavité centrale de la zone de capteurs (15).

6. Appareil, selon la revendication 4 ou 5,
**caractérisé en ce que**
la zone de capteurs (15) est en forme de plaque métallique pratiquement circulaire munie d'un anneau d'isolation (6) et installée dans la plaque chauffante (4).

7. Appareil, selon l'une des revendications 4 à 6,
**caractérisé en ce que**
la zone de capteurs (15) est installée avec un faible débordement dans la plaque chauffante (4) et le capteur thermique (9) est un composant optoélectronique fonctionnant selon le principe de l'écho-sonde.

8. Appareil, selon l'une des revendications 4 à 7,
**caractérisé par**
des thermostats de sécurité (12) installés sur la face arrière de la zone de capteurs (15) pour commander une installation de chauffage (14) et la couper en cas de surchauffe.

9. Appareil, selon la revendication 8,
**caractérisé en ce que**
l'installation de chauffage (14) est composée de feuilles chauffantes appliquées sur la face arrière de la plaque chauffante (4), deux feuilles chauffantes (14.1, 14.2) étant prévues de part et d'autres de la zone du capteur (15) et au moins une autre feuille chauffante est prévue au-dessus de la zone de capteur (15) et notamment transversalement aux deux feuilles chauffantes latérales (14.1, 14.2).

10. Appareil, selon l'une des revendications précédentes,
**caractérisé en ce que**
la température du liquide dans la poche de liquide d'irrigation (8) est réglable à la température du corps humain et il est prévu un système avertisseur mis en oeuvre en cas de surchauffe du liquide d'irrigation.

11. Appareil, selon l'une des revendications précédentes,
**caractérisé par**
un indicateur optique de température (35) comportant des diodes LED en forme de barres (36) ainsi qu'un marquage de la plage de consigne (37).

12. Appareil, selon l'une des revendications précédentes,
**caractérisé en ce que**
la partie inférieure (10) de l'appareil comporte une pompe (20), des indicateurs ou afficheurs (28, 29, 30) et des touches (26), les touches (26) étant des touches de sélection de la pression pour un procédé de basculement servant à vider la pompe (20) et le système de tuyaux (18, 22).

13. Appareil, selon l'une des revendications précédentes,
**caractérisé en ce que**
la plaque chauffante (4) est en aluminium.
